# EUROPÄISCHE PATENTSCHRIFT

(11) **EP 3 723 669 B1**
(45) Veröffentlichungstag und Bekanntmachung des Hinweises auf die Patenterteilung: **23.03.2022**
(21) Anmeldenummer: 18836353.5
(22) Anmeldetag: 13.12.2018
(51) Int. Cl.: A61F 2/58, A61F 2/68, A61F 2/70, A61F 2/72

(54) **PROTHESENHAND**
PROSTHETIC HAND
PROTHÈSE DE MAIN

(30) Priorität: 15.12.2017 DE 102017130082
(43) Veröffentlichungstag der Anmeldung: 21.10.2020
(73) Patentinhaber: Ottobock SE & Co. KGaA, 37115 Duderstadt (DE)
(72) Erfinder: BERTELS, Thomas, 37115 Duderstadt (DE); BRÜNJES, Lukas, 37075 Göttingen (DE); BIERBAUM, Sarah, 37073 Göttingen (DE)
(74) Vertreter: Gramm, Lins & Partner Patent- und Rechtsanwälte PartGmbB
(86) Internationale Anmeldenummer: PCT/EP2018/084670
(87) Internationale Veröffentlichungsnummer: WO 2019/115665

(56) Entgegenhaltungen:
- EP-A1- 0 045 818
- EP-B1- 0 045 818
- WO-A1-2012/071343
- WO-A2-2007/076765
- DE-A1-102014 001 390
- DE-U1-202016 003 671

## Beschreibung

Die Erfindung betrifft eine Prothesenhand mit einem Daumenelement und wenigstens einem Fingerelement, wobei das Daumenelement durch eine Steuerkraft relativ zu dem wenigstens einen Fingerelement bewegbar ausgebildet ist.

Derartige Prothesenhände sind aus dem Stand der Technik seit langem bekannt. Aus den EP 0 045 818 A1 und der WO 2007/076765 A2 sind Handprothesen mit beweglichen Fingergliedern bekannt. Durch die Bewegbarkeit des Daumenelementes relativ zum Fingerelement lässt sich die Hand öffnen, so dass beispielsweise Gegenstände gegriffen werden können oder sich der Träger der Prothesenhand beispielsweise an einer Stange oder einem Haltegriff festhalten kann. Bei einer derartigen Prothesenhand ist das Daumenelement herkömmlicherweise federbelastet ausgebildet, so dass es nach Wegfall der Steuerkraft wieder in die ursprüngliche Position, die regelmäßig die geschlossene Position ist, zurückkehrt. Durch die aufgebrachte Steuerkraft wird die Hand folglich geöffnet, durch ein Absenken oder Wegfallen der Steuerkraft wird sie wieder geschlossen. Es sind jedoch auch Prothesenhände bekannt, bei denen durch eine aufgebrachte Steuerkraft die Hand geschlossen wird und die nach Wegfall der Steuerkraft in die geöffnete Position zurückkehren.

Handprothesen können motorbetrieben sein, wobei insbesondere unterschiedliche Fingerelemente und das Daumenelement separat angetrieben und bewegt werden können. Derartige Handprothesen sind schwer, aufwendig und kostenintensiv. In alternativen Ausgestaltungen der Handprothesen, die nicht motorbetrieben sein müssen, wird die Steuerkraft durch ein Zugkraftelement ausgeübt, das beispielweise entlang des Armes des Trägers über die Schulter oder den Rumpf geführt wird. Durch eine Bewegung der Schulter auf der gegenüberliegenden Seite der durch die Prothesenhand versorgten oberen Gliedmaße wird die Steuerkraft aufgebracht und die Hand geöffnet oder geschlossen.

Mit einer Prothesenhand der eingangs erwähnten Art kann folglich ein Gegenstand gegriffen werden. Dabei wird häufig der sogenannte Oppositionsgriff verwendet, bei dem der Gegenstand zwischen der Fingerspitze des wenigstens einen Fingerelementes und der Spitze des Daumenelementes gegriffen wird. Dies ist jedoch für eine Vielzahl von Handhabungen unterschiedlicher Gegenstände, beispielsweise eines Schlüssels, unpraktisch. Mit einer gesunden natürlichen Hand würde der Griff gewechselt und der sogenannte Lateralgriff verwendet werden. Dabei wird der zu greifende Gegenstand zwischen dem Daumen und der Seitenkante des Zeigefingers eingeklemmt. Es gibt zwar motorbetriebene Prothesenhände, bei denen über Steuersignale von einem in den anderen Griffmodus umgeschaltet werden kann, für nichtmotorbetriebene Prothesenhände sind Umschaltungen der Daumenposition aus dem Stand der Technik nicht bekannt. Eine solche Prothesenhand ist aus der DE 20 2016 003 671 U1, aus der DE 10 2014 001 390 A1 und aus der WO 2012/071343 A1 bekannt. Der vorliegenden Erfindung liegt daher die Aufgabe zugrunde, eine Prothesenhand gemäß dem Oberbegriff des Anspruchs 1 so weiterzuentwickeln, dass unterschiedliche Griffmodi realisiert werden und eine intuitive und einfache Umschaltung zwischen den einzelnen Modi möglich ist.

Die Erfindung löst die gestellte Aufgabe durch eine Prothesenhand mit einem Daumenelement und wenigstens einem Fingerelement, wobei das Daumenelement durch eine Steuerkraft relativ zu dem wenigstens einen Fingerelement bewegbar ausgebildet ist, wobei sich die Prothesenhand dadurch auszeichnet, dass das Daumenelement in wenigstens einem ersten Modus entlang einer ersten Trajektorie und in wenigstens einem zweiten Modus entlang einer zweiten Trajektorie relativ zu dem wenigstens einen Fingerelement bewegbar ist und von dem ersten Modus in den zweiten Modus bringbar ist, indem die Steuerkraft einen ersten vorbestimmten Grenzwert übersteigt. Vorzugsweise kann das Daumenelement in mehr als zwei Modi, insbesondere drei oder vier Modi bewegt werden. Dabei wird das Daumenelement von einem Modus in einen anderen Modus gebracht, indem die Steuerkraft einen vor diesen Moduswechsel vorbestimmten Grenzwert überschreitet. Dieser Grenzwert kann für alle Moduswechsel gleich oder individuell verschieden gewählt sein.

Vorteilhafterweise ist das Daumenelement von dem zweiten Modus in den ersten Modus bringbar, indem die Steuerkraft einen zweiten vorbestimmten Grenzwert überschreitet. Dabei hat es sich als vorteilhaft herausgestellt, wenn der erste vorbestimmte Grenzwert identisch zum zweiten vorbestimmten Grenzwert ist.

Mit einer Handprothese kann der Träger folglich einfach zwischen den beiden Greifmodi umschalten, indem die ohnehin aufzubringende Steuerkraft vergrößert wird. Vorteilhafterweise verfügt die Prothesenhand über eine mechanische Anordnung, die in einen Totpunkt oder über einen Totpunkt hinweg bewegt wird, indem die Steuerkraft den vorbestimmten Grenzwert übersteigt.

Vorteilhafterweise verfügt die Prothesenhand über wenigstens ein Kraftaufbringelement, das eine Rückstellkraft auf das Daumenelement und vorzugsweise auf das wenigstens eine Fingerelement aufbringt, die der Steuerkraft entgegengerichtet ist. Ein solches Kraftaufbringelement kann ein Federelement, beispielsweise eine Torsionsfeder, eine Konstantkraftfeder oder eine Schraubenfeder sein. Dadurch wird sichergestellt, dass die Stellung der Prothesenhand ohne durch den Träger aufgebrachte Steuerkraft der geschlossene Zustand ist. Je nach Modus, in dem sich das Daumenelement befindet, ist diese Endstellung bevorzugt entweder der Oppositionsgriff oder der Lateralgriff.

Die Rückstellkraft ist bevorzugt derart gewählt, dass sie das Daumenelement ohne Steuerkraft im ersten Modus in eine erste Endposition und im zweiten Modus in eine zweite Endposition bringt, die von der ersten Endposition verschieden ist. Dabei handelt es sich bevorzugt um die bereits genannten Griffmodi "Oppositionsgriff" und "Lateralgriff". In diesen Fällen liegt das Daumenelement in der ersten Endposition und/oder in der zweiten Endposition an dem wenigstens einen Fingerelement an. Dabei ist zu beachten, dass das Daumenelement in den unterschiedlichen Endpositionen an unterschiedlichen Teilen des Fingerelementes, beispielsweise einer Fingerkuppe oder einer Seitenfläche des Fingerelementes, anliegt.

Vorteilhafterweise verfügt die Prothesenhand über wenigstens ein Zugelement, durch das die Steuerkraft aufbringbar ist. Dieses Zugelement ist vorteilhafterweise an der Schulter, vorzugsweise an der der Prothesenhand gegenüberliegenden Schulter, oder einem Rumpf des Trägers der Prothesenhand angeordnet.

Das Daumenelement wird im ersten Modus entlang einer ersten Trajektorie bewegt, wenn eine Steuerkraft aufgebracht wird. Solange die Steuerkraft den vorbestimmten ersten Grenzwert nicht überschreitet, verbleibt das Daumenelement im ersten Modus und die Hand wird durch die Steuerkraft geöffnet und durch Nachlassen oder Entfallen der Steuerkraft entlang der ersten Trajektorie geschlossen. Überschreitet sie jedoch den ersten vorbestimmten Grenzwert, wird das Daumenelement oder ein mit ihm verbundenes Element vorzugsweise über einen Totpunkt der Bewegung hinweg bewegt und folgt nun der zweiten Trajektorie, sobald die Steuerkraft nachlässt. Wird die Steuerkraft wieder erhöht, ohne den zweiten vorbestimmten Grenzwert zu überschreiten, wird die Hand erneut geöffnet und beim Nachlassen oder Wegfallen der Steuerkraft wieder entlang der zweiten Trajektorie geschlossen. Dadurch wird die Hand in dem zweiten Modus, beispielsweise ein anderer Griffmodus, verwendet. Wird die Steuerkraft über den zweiten vorbestimmten Grenzwert erhöht, wird das Daumenelement oder ein mit ihm verbundenes Element erneut über den Totpunkt der Bewegung hinweg bewegt, der der gleiche oder ein anderer Totpunkt sein kann. Wird die Steuerkraft anschließend reduziert oder entfällt vollständig, wird die Hand erneut geschlossen und das Daumenelement bewegt sich erneut entlang der ersten Trajektorie im ersten Modus.

Vorzugsweise ist das Daumenelement in wenigstens einem dritten Modus entlang einer dritten Trajektorie und vorzugsweise in wenigstens einem vierten Modus entlang einer vierten Trajektorie bewegbar.

Das Zugelement ist in einer besonderen Ausführungsform durch einen Aktuator betätigbar. Dies kann beispielsweise ein motorgetriebener Aktuator sein, der ein Betätigungselement aufweist.

Alternativ zu dem Umschalten zwischen unterschiedlichen Bewegungsmodi dadurch, dass eine Steuerkraft einen vorbestimmten Grenzwert übersteigt, kann auch eine Fliehkraftkupplung oder eine ähnliche Ausgestaltung vorgesehen sein, bei der das Umschalten zwischen mehreren Bewegungsmodi dann geschieht, wenn eine Bewegung eines Aktuators, beispielsweise eines Zugseils, eine Geschwindigkeit aufweist, die einen vorbestimmten Grenzwert überschreitet. Die verschiedenen Ausgestaltungen können mutatis mutandis angewandt werden. Auch hier ist es möglich, mehr als zwei Bewegungsmodi vorzusehen. Das Umschalten zwischen unterschiedlichen Bewegungsmodi erfordert das Überschreiten vorbestimmter Grenzwerte, die je nach Modus, in dem geschaltet werden soll, unterschiedlich oder identisch ausgebildet werden können.

Anhand der beiliegenden Zeichnungen werden nachfolgend einige Ausführungsbeispiele der vorliegenden Erfindung näher erläutert. Es zeigt:
- Figur 1 -: die schematische dreidimensionale Ansicht eines Teils einer Prothesenhand,
- Figur 2 -: die Darstellung aus Figur 1 in einer Draufsicht,
- Figur 3 -: die schematische Ansicht eines Teils einer Prothesen-hand gemäß einem weiteren Ausführungsbeispiel der vorliegenden Erfindung,
- Figuren 4a und 4b -: weitere schematische Ansichten eines Teils einer Pro-thesenhand,
- Figur 5 -: die schematische Darstellung eines Teils eines weite-ren Ausführungsbeispiels der vorliegenden Erfindung,
- Figuren 6a bis 6e -: verschiedene Stadien beim Umschalten der Vorrichtung aus Figur 5,
- Figur 7 -: die schematische Darstellung eines Teils einer weiteren Ausführungsform der vorliegenden Erfindung,
- Figur 8a und 8b -: schematische Teilansichten der Vorrichtung aus Figur 7,
- Figur 9 -: die schematische Darstellung eines Teils einer weiteren Ausführungsform,
- Figur 10a bis 10c -: schematische Teilansichten der Figur 9,
- Figur 11 -: eine schematische Schnittdarstellung durch ein Teil der Vorrichtung aus Figur 9,
- Figuren 12, 13 und 14 -: schematische Darstellungen unterschiedlicher Bewegungszustände und
- Figur 15 -: die schematische Darstellung einer Handprothese im getragenen Zustand.

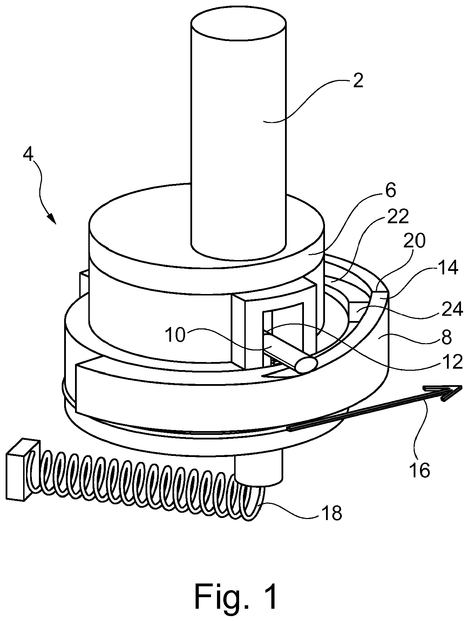

Figur 1 zeigt die schematische dreidimensionale Ansicht eines Teils einer Prothesenhand gemäß einem ersten Ausführungsbeispiel der vorliegenden Erfindung. Ein Teil eines Daumenelementes 2 ist auf einem drehbaren Gestell 4 mit einer Drehplatte 6 angeordnet, die sich relativ zu einer ortsfesten Profilscheibe 8 drehen kann. Seitlich zu der Drehplatte 6 ist ein Führungspin 10 angeordnet, der durch wenigstens eine Feder 12 nach radial außen und unten gedrückt wird. Er gleitet auf einer Führungskontur 14 auf der Profilscheibe 8 entlang. Entlang des Pfeils 16 kann die Steuerkraft ausgeübt werden. Diese wird auf das Gestell 4 und damit auf die Drehplatte 6 den Führungspin 10 und das Daumenelement 2, nicht jedoch auf die Profilscheibe 8 ausgeübt. Für ein Kraftaufbringelement 18, das im gezeigten Ausführungsbeispiel als Schraubenfeder ausgebildet ist, wird eine Rückstellkraft ausgeübt. Die Richtung der Steuerkraft und damit die Richtung des Pfeiles 16 kann sich im Verlauf der Bewegung ändern. dies wird vorzugsweise durch eine flexible unelastische Anbindung erreicht, die an der gleichen Stelle am Umfang der Drehplatte 6 angreift, an der sich auch der Führungspin 10 befindet.

Wird nun entlang des Pfeils 16 eine Steuerkraft ausgeübt, die kleiner ist als ein vorbestimmter Grenzwert, wird die Drehplatte 6 und das Daumenelement 7 relativ zur Profilscheibe 8 gedreht und der Führungspin 10 gleitet auf der Führungskontur 14 ab. Dabei wird die wenigstens eine Feder 12 in vertikaler Richtung gespannt Die Führungskontur 14 verfügt über einen Maximalpunkt 20, der durch eine Steuerkraft, die kleiner ist als der erste vorbestimmte Grenzwert, nicht erreicht wird. Wird die Steuerkraft 16 reduziert oder vollständig weggelassen, sorgt die durch das Kraftaufbringelement 18 aufgebrachte Rückstellkraft dafür, das Daumenelement 2 mit dem Gestell 4 wieder in die Ausgangsposition zurückzudrehen. Wird jedoch die Steuerkraft so gewählt, dass sie größer ist als der vorbestimmte Grenzwert, wird der Führungspin 10 den Maximalpunkt 20 erreichen. Durch die vertikal wirkende Federkraft auf den Führungspin 10 gleitet dieser der Schräge um den Maximalpunkt 20, der ein Totpunkt ist, ab und überschreitet damit diesen Totpunkt. Die durch das Kraftaufbringelement 18 aufgebrachte Rückstellkraft sorgt dafür, dass eine weitere Drehbewegung entgegen dem Uhrzeigersinn stattfindet, die das Gestell 4 mit dem Daumenelement 2 in die andere Endposition bringt.

Wird erneut eine Steuerkraft entlang der Pfeilrichtung 16 aufgebracht, kommt es zu einer Drehung des Daumenelementes 2 und des Gestells 4 im Uhrzeigersinn. Die radial äußere Führungskontur 14 ist im nicht dargestellten hinteren Bereich in radialer Richtung derart ausgebildet, dass der Führungspin 10 entgegen der durch die Feder 12 aufgebrachte Federkraft in die Drehplatte 6 gedrückt wird. Dadurch gleitetder Führungspin 10 auf der radial inneren Führungskontur 22 ab, wobei die Feder 12 wieder in vertikaler Richtung gespannt wird. Auch die Führungskontur 22 verfügt über einen Maximalpunkt 24, über den der Führungspin 10 unterstützt von der in vertikaler Richtung wirkenden Federkraft der Feder 12 hinweggleitet, wenn eine Steuerkraft aufgebracht wird, die größer ist als der zweite vorbestimmte Grenzwert.

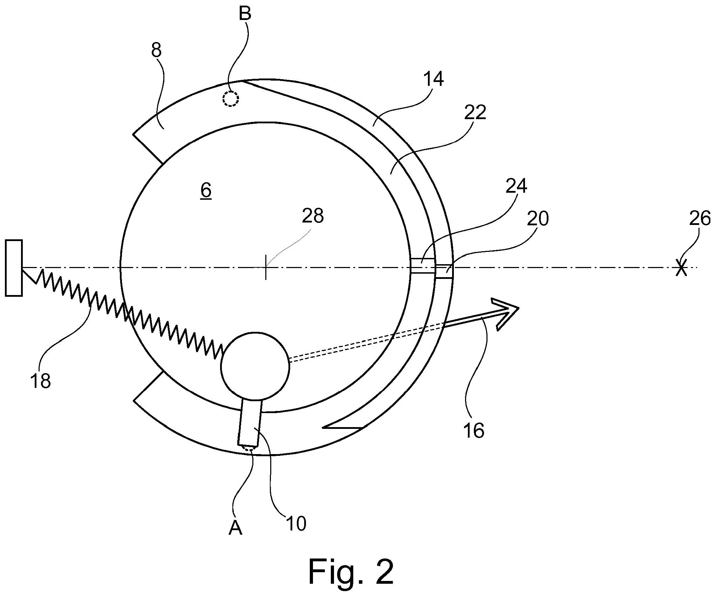

Figur 2 zeigt die Darstellung schematisch von oben. Man erkennt das Daumenelement 2, die Drehplatte 6 sowie das Kraftaufbringelement 18 und die Profilscheibe 8. Sie verfügt über die radial äußere Führungskontur 14 und die radial innere Führungskontur 22. Beide verfügen jeweils über einen Maximalpunkt 20, 24. Der Führungspin 10 befindet sich im gezeigten Ausführungsbeispiel an einem ersten Endpunkt der Bewegungskontur. Durch die entlang des Pfeils 16 aufzubringende Steuerkraft wird die Drehplatte 6 um die Rotationsachse 28 gedreht und der Führungspin 10 gleitet auf der äußeren Führungskontur 14 ab. Ist die Steuerkraft größer als der erste vorbestimmte Grenzwert, wird der Maximalpunkt 24 der äußeren Führungskontur 14 erreicht und die durch die Feder 12 aufgebrachte Federkraft und die Rückstellkraft, die durch das Kraftaufbringelement 18 aufgebracht wird, sorgen dafür, dass der Führungspin 10 zum entgegengesetzten zweiten Endpunkt weitergleitet. Man erkennt, dass die äußere Führungskontur 14 in diesem Bereich radial nach außen verjüngt ist. Wird erneut eine Steuerkraft aufgebracht, gleitet der Führungspin 10 entlang der radial inneren Führungskontur 22 und die Hand öffnet und schließt im zweiten Modus, solange der zweite vorbestimmte Grenzwert nicht überschritten wird.

Die Richtung der Steuerkraft, die durch den Pfeil 16 visualisiert wird, kann sich im Verlauf der Bewegung ändern, durchläuft bevorzugt jedoch immer den Punkt 26.

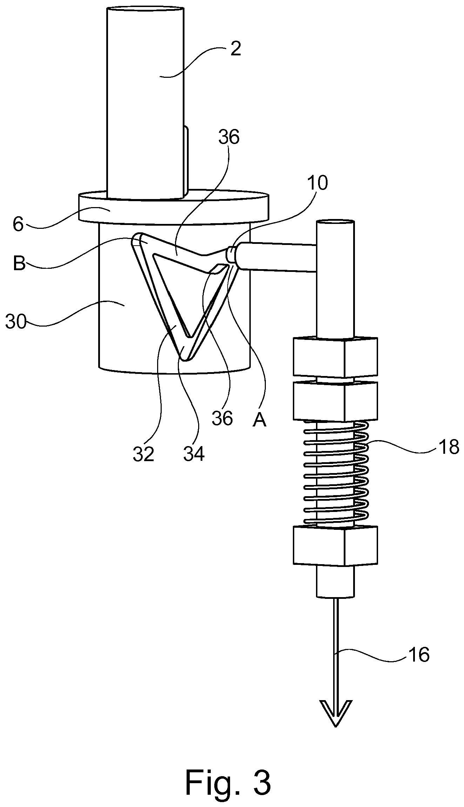

Figur 3 zeigt eine alternative Ausgestaltung. Auch hier ist das Daumenelement 2 auf einer Drehplatte 6 angeordnet. Darunter befindet sich ein Zylinder 30, in dem sich eine herzförmige Nut 32 befindet.

Die Steuerkraft kann wieder entlang des Pfeils 16 aufgebracht werden. Dadurch wird der Führungspin 10 in der Nut 32 aus der Ruheposition A nach unten verschoben wobei er die Drehplatte 6 gegen den Uhrzeigersinn rotiert. Ist die Steuerkraft 16 kleiner als der erste vorbestimmte Grenzwert, wird beim Nachlassen der Steuerkraft der Führungspin 10 durch das Kraftaufbringelement 18 wieder in die in Figur 3 gezeigte Position A zurückbewegt und verbleibt so im gleichen Griffmodus.

Ist die Steuerkraft größer als der vorbestimmte Grenzwert, wird der Führungspin 10 in den Minimalpunkt 34 gezogen und wird sich beim Reduzieren der Steuerkraft entlang eines linken Anteils der Nut 32 bewegen, wobei die Drehplatte 6 weiter gegen den Uhrzeigersinn rotiert wird bis der zweite Endpunkt B erreicht ist, der einem zweiten Griffmodus entspricht.

Aus dem zweiten Endpunkt B heraus wird sich der Führungspin 16 beim Auftreten einer Steuerkraft entlang des Pfeils 16 entlang des oberen linken Bogens 36 der herzförmigen Nut 32 bewegen, wobei die Drehplatte 6 im Uhrzeigersinn gedreht wird. Wird die Kraft vor dem Erreichen des Minimalpunktes 36 wieder reduziert, verbleibt die Hand im zweiten Griffmodus und erreicht gesteuert durch die Rückstellkraft des Kraftaufbringelementes 18 in Punkt B die Ruheposition. Erst bei Erreichen des Minimalpunkts 36 (oberes Minimum) wird in den ersten Modus zurückgeschaltet, indem die Drehplatte 6 weiter im Uhrzeigersinn gedreht und die Endposition A erreicht wird.

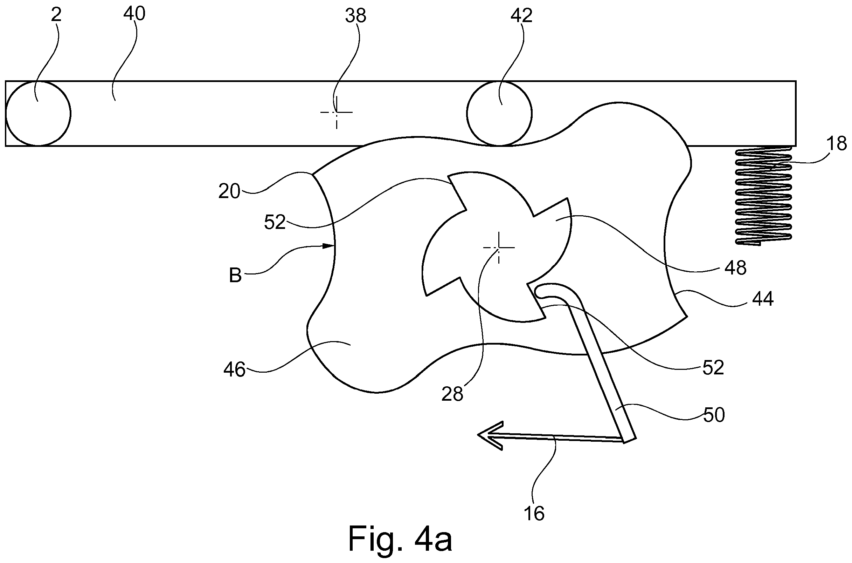

Figur 4a zeigt eine weitere Ausgestaltung eines Teils der Handprothese. Das Daumenelement 2 ist in diesem Fall an einem um eine Schwenkachse 38 schwenkbar gelagerten Hebel 40 angeordnet, an dessen dem Daumenelement 2 entgegengesetztem Ende das Kraftaufbringelement 18 in Form einer Zugfeder angeordnet ist. Der Hebel 40 verfügt über einen Nocken 42, der an einer Außenkontur 44 einer Kurvenscheibe 46 abgleitet. Die Kurvenscheibe 46 ist mit einem Ratschenelement 48 drehfest verbunden. Beide Elemente zusammen können um die Rotationsachse 28 gedreht werden. Dazu ist ein Hebel 50 vorhanden, auf den entlang des Pfeils 16 die Steuerkraft ausgeübt wird. Da das Ende des Hebels 50 mit den Vorsprüngen 52 des Ratschenelementes 48 zusammenwirkt, führt die Steuerkraft entlang des Pfeils 16 zu einer Rotation des Ratschenelementes 48 und der Kurvenscheibe 46, so dass sich die Position des Nockens 42 an der Außenkontur 44 verändert. Dies führt zu einem Verschwenken des Hebels 40 um die Schwenkachse 38 und damit zu einem Bewegen des Daumenelementes 2.

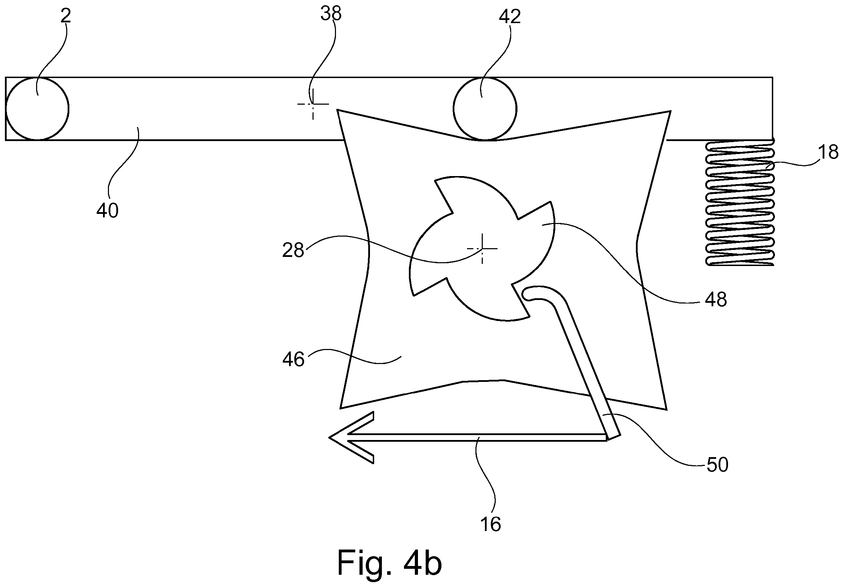

Ist die entlang des Pfeils 16 aufgebrachte Steuerkraft größer als ein vorbestimmter Grenzwert, wird dabei der Maximalpunkt 20 überschritten, so dass sich der Hebel 40 bei Entlastung nicht mehr in die in Figur 4 gezeigte Position zurückbewegt. Stattdessen zieht das Kraftaufbringelement 18 den Nocken 42 in die zweite Endposition B, die einem anderen Griffmodus entspricht.

Figur 4b zeigt eine ähnliche Ausgestaltung wie Figur 4a. Der Unterschied zwischen den beiden Figuren besteht in der Außenkontur 44 der Kurvenscheibe 46 und der Position der Rotationsachse 28. Während die Rotationsachse 28 in Figur 4a so angeordnet ist, dass jeweils zwei der möglichen Einstellungen, nämlich die entgegengesetzten Seiten der Kurvenscheibe 46, identisch ausgebildet sind und so zwei Einstellmöglichkeiten für zwei Modi vorhanden sind, ist die Rotationsachse 28 in Figur 4b nicht in der Mitte der Kurvenscheibe 46 angeordnet. Wird durch den Hebel 50 eine Steuerkraft entlang der Richtung des Pfeiles 16 aufgebracht, die größer ist als der vorbestimmte Grenzwert, wird das Ratschenelement 48 um 90° weiter gedreht. Damit kommt eine andere Seite der Außenkontur 44 der Kurvenscheibe 46 mit dem Nocken 42 in Kontakt. Da die unterschiedlichen Seiten der Außenkontur 44 von der Rotationsachse 28 unterschiedliche Abstände aufweisen, werden auf diese Weise vier unterschiedliche Betriebsmodi des Daumenelementes erreicht.

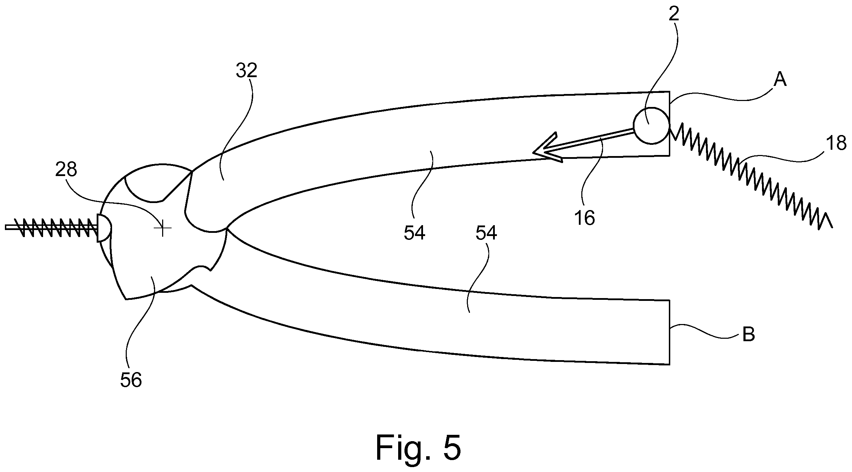

Bei der in Figur 5 gezeigten Möglichkeit kann das Daumenelement 2 mittels Steuerkraft 16 entgegen der Kraft eines Kraftaufbringelementes 18 in einer Nut 32 verschoben werden. Die Nut verfügt über zwei Äste 54, an deren Enden sich der erste Endpunkt A und der zweite Endpunkt B befinden. Zwischen den beiden Ästen 54 ist ein Umschaltelement 56 vorhanden, das um die Rotationsachse 28 drehbar gelagert ist.

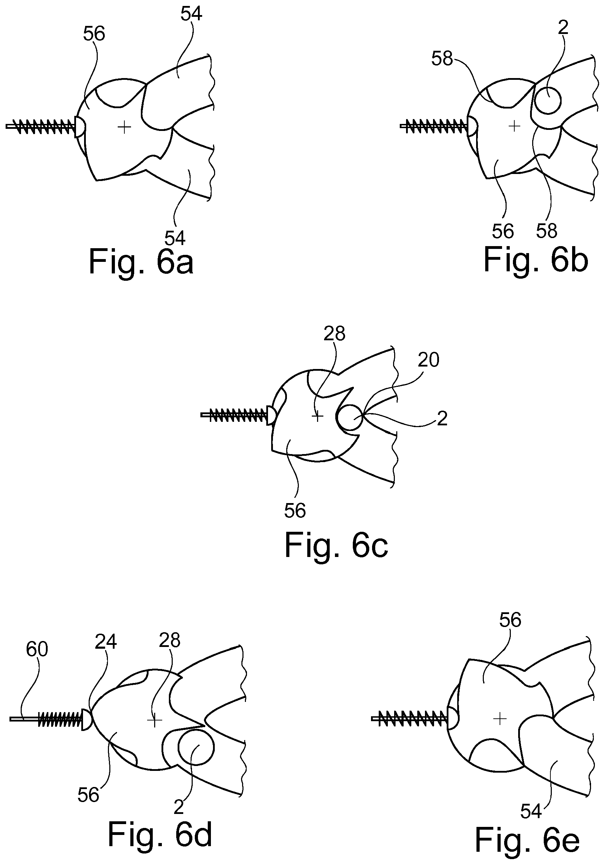

In den Figuren 6a bis 6e ist im Detail dargestellt, wie der Umschaltvorgang in diesem Fall stattfindet. Figur 6a zeigt die Situation der beiden Äste 54 der Nut 32 mit dem Umschaltelement 56 in der in Figur 5 gezeigten Position. Wird eine Steuerkraft auf das Daumenelement 2 ausgebildet, gelangt es in die in Figur 6b gezeigte Position. Das Umschaltelement 56 verfügt über zwei Vertiefungen 58, wobei das Daumenelement 2, gezogen durch die Steuerkraft 16, in eine dieser beiden Vertiefungen eingebracht wird. Durch eine weitere Erhöhung der Steuerkraft wird die in Figur 6c gezeigte Position erreicht. Das Daumenelement 2 hat den Maximalpunkt 20 der Nut überschritten und dabei das Umschaltelement 56 um die Rotationsachse 28 gedreht. Eine weitere Erhöhung führt zu der in Figur 6d gezeigten Situation. Das Umschaltelement 56 wurde weiter um die Rotationsachse 28 gedreht, bis auch ein federbelasteter Stößel 60, der an dem Umschaltelement 56 gleitend gelagert ist, dessen Maximalpunkt 24 überschreitet. Durch die von dem Federelement des federbelasteten Stößel 60 auf das Umschaltelement 56 ausgeübte Kraft wird das Umschaltelement 56 weiter um die Rotationsachse gedreht, bis es die in Figur 6e gezeigte zweite Endposition erreicht hat. Das Daumenelement 2 hat den gezeigten Bereich in dem unteren Ast 54 der Nut 32 verlassen und erreicht so die Endposition B.

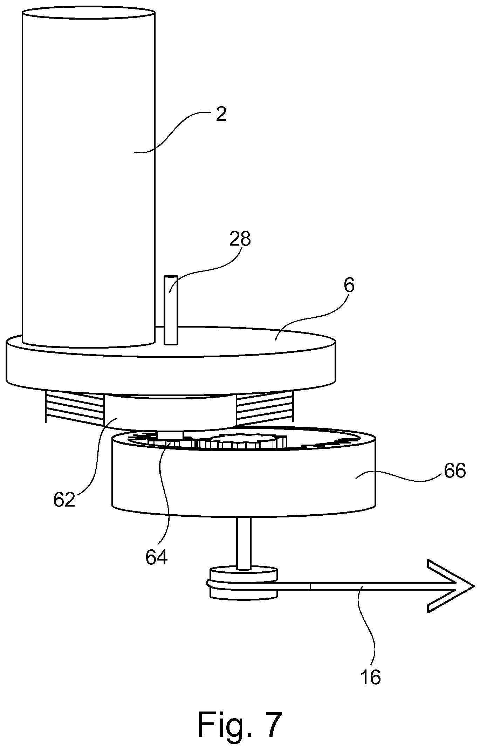

Ein weiteres Ausführungsbeispiel ist Figur 7 zu entnehmen. Das Daumenelement 2 ist wieder auf der Drehplatte 6 angeordnet, die um eine Rotationsachse 28 drehbar gelagert ist. Sie ist über eine Übertragungsscheibe 62, mit der die Drehplatte 6 drehfest gekoppelt ist, mit einem Zahnrad 64 verbunden, das in einer Antriebsscheibe 66 umläuft. Entlang des Pfeils 16 kann die Steuerkraft auf die Mechanik aufgebracht werden.

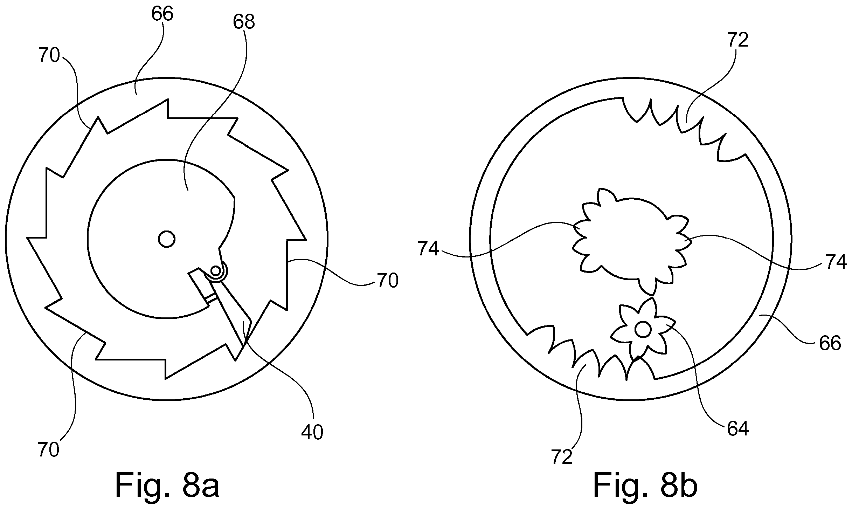

Figur 8a zeigt die Unterseite der Antriebsscheibe 66. Über eine Konturscheibe 68 wird die Steuerkraft aufgebracht. Der Hebel 40 wirkt mit den Zähnen 70 nach Art eines Freilaufes zusammen. Wird die Konturscheibe 68 durch die Steuerkraft im Uhrzeigersinn bewegt, führt dies zu einer Rotation der Antriebsscheibe 66. Wird hingegen die Steuerkraft abgesenkt oder vollständig abgestellt, sorgt eine durch ein nicht gezeigtes Kraftaufbringelement aufgebrachte Rückstellkraft dafür, dass die Konturscheibe 68entgegen dem Uhrzeigersinn gedreht werden kann, während die Antriebsscheibe 66 in Position bleibt.

Figur 8b zeigt eine Draufsicht auf die Antriebsscheibe 66. Man erkennt, dass sie zwei äußere Zahnbereiche 72 und zwei innere Zahnbereiche 74 aufweist. Das Zahnrad 64 greift je nach Modus, in dem Daumenelement betrieben wird, in den äußeren Zahnbereich 72 oder den inneren Zahnbereich 74 ein und wird durch eine Rotation der Antriebsscheibe 66 in Abhängigkeit vom eingreifenden Zahnbereich im Uhrzeigersinn oder entgegen dem Uhrzeigersinn gedreht und erreicht unterschiedliche Modi.

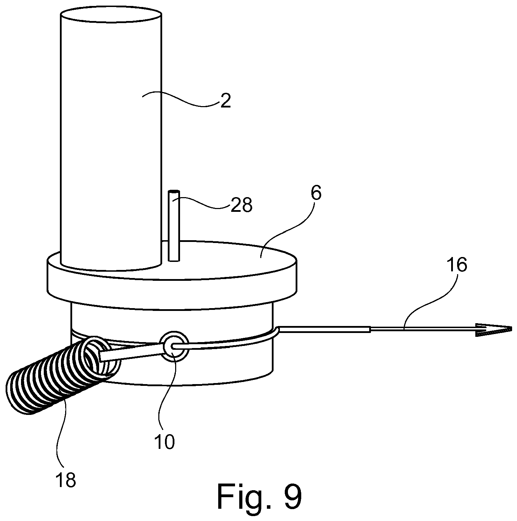

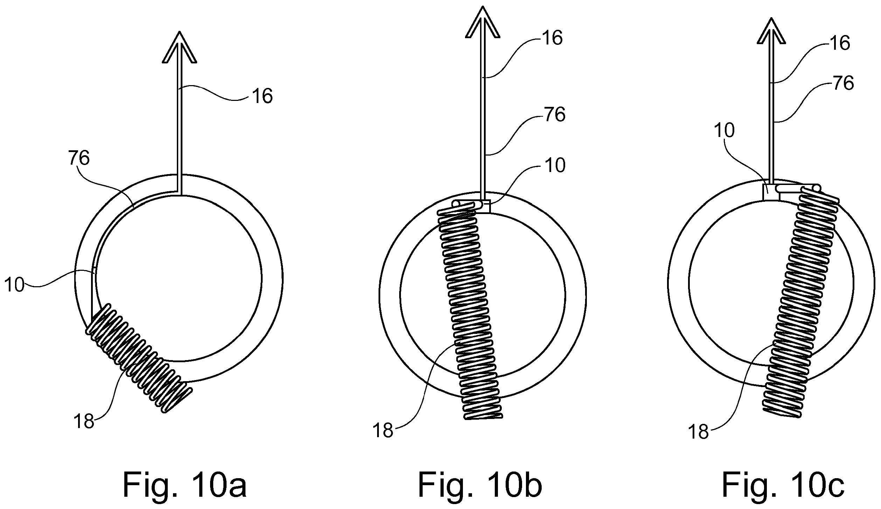

Die Figuren 9, 10a, 10b, 10c und 11 zeigen ein weiteres Ausführungsbeispiel, bei dem das Daumenelement 2 auf einer Drehplatte 6 um die Rotationsachse 28 herum drehbar gelagert ist. Die vom Kraftaufbringelement 18 aufgebrachte Rückstellkraft wirkt auf den Führungspin 10, auf den auch entlang des Pfeils 16 die Steuerkraft aufgebracht werden kann. Figur 10a und 10b zeigen die schematische Situation von unten. Der Führungspin 10 ist in einer nicht dargestellten Ausnehmung der Drehplatte 6 enthalten. Die durch das Kraftaufbringelement 18 aufgebrachte Rückstellkraft wirkt auf den Führungspin 10. Entlang des Pfeils 16 wird die Steuerkraft über einen Zugseil 76 aufgebracht. Ist die Steuerkraft geringer als der vorbestimmte Grenzwert, kommt es nicht zu der in Figur 10b gezeigten maximalen Rotation, sondern der Führungspin 10 wird maximal bis zu einer Position zwischen den in den Figuren 10a und 10b gezeigten Position verdreht. Beim Nachlassen der Steuerkraft wird durch die Rückstellkraft 18 wieder die in Figur 10a gezeigte erste Endposition erreicht. Ist die entlang des Pfeils 16 aufgebrachte Steuerkraft jedoch größer als der vorbestimmte Grenzwert, wird die in Figur 10b gezeigte maximale Verschiebeposition des Führungspins 10 erreicht. In diesem Fall erstreckt sich der Zugseil 76 und der Pfeil 16 parallel zur Längsrichtung des Führungspins 10, der somit aus der Ausnehmung zumindest ein Stück herausgezogen wird.

Figur 10c zeigt die Situation, in der die entlang der Richtung des Pfeils 16 aufgebrachte Steuerkraft den vorbestimmten ersten Grenzwert überschritten hat. Man erkennt, dass die Rückstellkraft 18 nun den Führungspin 10 in den rechten Teil der kreisförmigen Führung ziehen wird, so dass das Daumenelement im zweiten Modus betrieben wird.

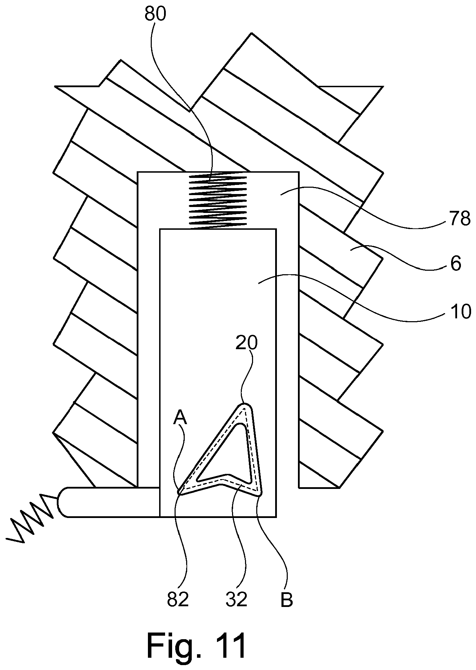

Figur 11 zeigt eine vergrößerte Schnittdarstellung durch die Drehplatte 6, in der sich die Ausnehmung 78 befindet, in der der Führungspin 10 angeordnet ist. Durch das Federelement 80 wird der Führungspin 10 in die Ausnehmung 78 gezogen. Man erkennt am Führungspin eine herzförmige Nut 32, die in Form und Ausgestaltung der in Figur 3 zeigten Nut entspricht. An dem aufgrund der Schnittdarstellung nicht dargestellten Anteil der Drehplatte 6 befindet sich ortsfest der schematisch angedeutete Steuerpin 82, der in der herzförmigen Nut 32 positioniert ist. Er befindet sich in Figur 11 im ersten Endpunkt A, so dass sich der Angriffspunkt der Rückstellkraft, die durch das Kraftaufbringelement 18 aufgebracht wird, in dieser Ansicht links der Rotationsachse 28 der Drehplatte 6 befindet. Wird nun die in Figur 10b gezeigte maximale Rotationsposition erreicht und der Führungspin aus der Ausnehmung 78 nach radial außen gezogen, rotiert er durch die Verschiebung des Steuerpins 82 in der Nut 32.

Beim Nachlassen der Steuerkraft vor Erreichen des Maximalpunktes 20 wird der Steuerpin 82 durch die Feder 80 zurück in die Position A gezogen. Durch eine Steuerkraft 16, die einen vorbestimmten Grenzwert erreicht oder überschreitet, wird der Führungspin 10 so weit gezogen und entsprechend rotiert, dass der Steuerpin 82 den Maximalpunkt 20 erreicht. Wird dann die Steuerkraft 16 reduziert, zieht die Feder 80 den Führungspin 82 zurück in die Ausnehmung 78, wobei der Steuerpin 82 die rechte dargestellte Bahn durchläuft, bis er die Endposition B erreicht. Der Führungspin 82 ist dabei so weit rotiert worden, dass der Angriffspunkt der Rückstellkraft, die durch das Kraftaufbringelement 18 aufgebracht wird, nun rechts der Rotationsachse 28 der Drehplatte 6 liegt und die Drehplatte 6 entsprechend in die andere Richtung gedreht wird, wodurch ein alternative Daumenposition erreicht wird.

Die gezeigten Ausführungsbeispiele sind lediglich zur Illustration und nicht beschränkend auszulegen. Selbstverständlich sind unterschiedlichste Formen der gezeigten Nuten, Führungen oder Kulissen möglich, um den gewünschten Effekt zu erreichen.

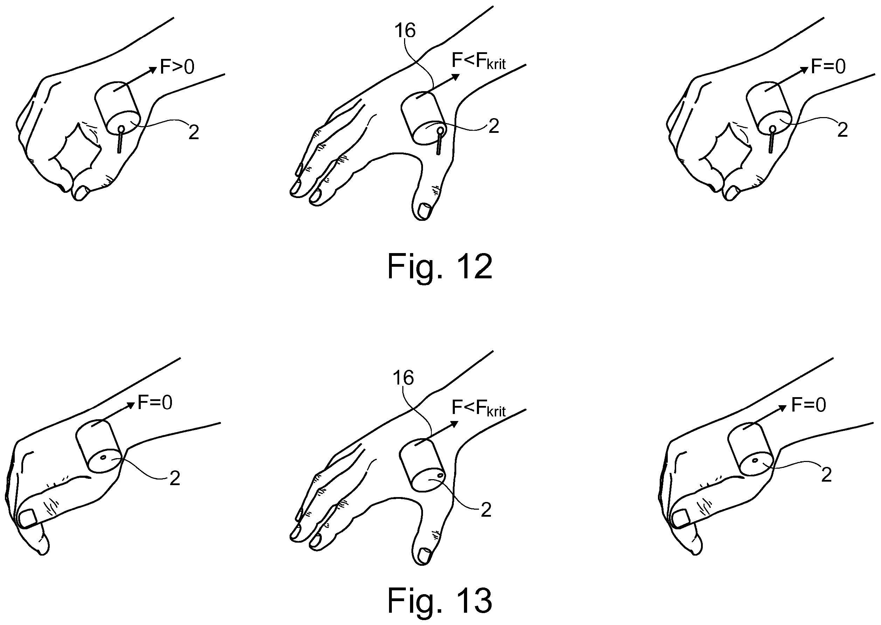

Die Figuren 12, 13 und 14 zeigen in jeweils drei nebeneinander angeordneten Darstellungen unterschiedliche Zustände einer Prothesenhand. In Figur 12 ist in der linken Darstellung die Hand im sogenannten Oppositionsgriff dargestellt, bei der die Daumenspitze an der Spitze des Zeigefingers anliegt. Schematisch in die Hand eingezeichnet ist das Daumenelement 2. In der linken Darstellung wird keine Steuerkraft ausgeübt, so dass die Hand geschlossen ist. In der mittleren Darstellung der Figur 12 hingegen wird eine Steuerkraft 16 auf das Daumenelement 2 ausgeübt, so dass die Hand geöffnet wird. Da die Steuerkraft in diesem Fall jedoch kleiner ist, als der vorbestimmte Grenzwert, kommt es nicht zu einem Moduswechsel. Wird die Steuerkraft 16 reduziert oder vollständig entfernt, stellt sich die in Figur 12 rechts dargestellte Situation ein, in der sich die Hand noch immer im Oppositionsgriff befindet.

Figur 13 zeigt in der linken Darstellung ebenfalls eine Hand mit einem Daumenelement 2, auf das keine Steuerkraft 16 ausgeübt wird. Anders als in Figur 12 befindet sich die Hand in Figur 13 jedoch im sogenannten Lateralgriff, so dass der Daumen an der Seitenfläche des Zeigefingers anliegt. In der mittleren Darstellung der Figur 13 wird eine Steuerkraft 16 auf das Daumenelement 2 ausgeübt, so dass die Hand geöffnet wird. Auch in diesem Fall ist die Steuerkraft 16 jedoch kleiner als der vorbestimmte Grenzwert, so dass sich beim Reduzieren und Entfernen der Steuerkraft 16 die Hand wieder schließt und die sich in Figur 13 rechts dargestellte Situation einstellt. Die Hand ist geschlossen und befindet sich immer noch im Lateralgriff.

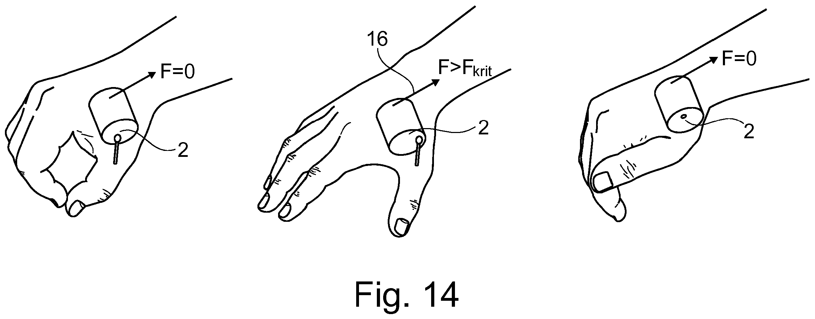

Figur 14 zeigt in ihrer linken Darstellung eine geschlossene Hand im Oppositionsgriff. Eine Steuerkraft wird auf das Daumenelement 2 nicht ausgeübt. In der mittleren Darstellung wird die Hand durch die Steuerkraft 16 geöffnet. Die Steuerkraft überschreitet in diesem Fall den vorbestimmten Grenzwert, so dass es zu einem Moduswechsel der Hand kommt. Wird die Steuerkraft 16 wieder reduziert oder vollständig entfernt, stellt sich die in Figur 14 rechts dargestellte Situation ein, in der die Hand zwar geschlossen ist, sich jedoch nicht mehr im Oppositionsgriff, sondern im Lateralgriff befindet. Ein Moduswechsel hat stattgefunden.

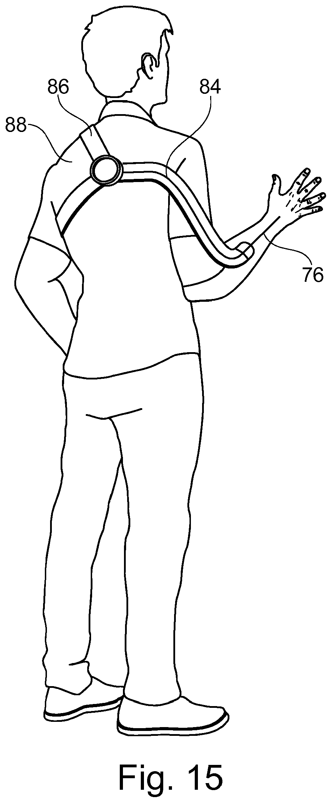

Figur 15 zeigt einen Träger einer Prothesenhand gemäß einem Ausführungsbeispiel der vorliegenden Erfindung. Über ein Zugseil 76 kann eine Zugkraft ausgeübt werden, die als Steuerkraft 16 wirkt. An dem Zugseil 76 befindet sich ein Zugriemen 84, der über eine Schulterschlaufe 86 an einer Schulter 88 des Trägers angeordnet ist. So kann der Träger durch eine Bewegung der Schulter 88 die nötige Zugkraft aufbringen.

### Bezugszeichenliste

- A: erster Endpunkt
- B: zweiter Endpunkt
- 2: Daumenelement
- 4: Gestell
- 6: Drehplatte
- 8: Profilscheibe
- 10: Führungspin
- 12: Feder
- 14: Führungskontur
- 16: Pfeil (Steuerkraft)
- 18: Kraftaufbringelement
- 20: Maximalpunkt
- 22: innere Führungskontur
- 24: Maximalpunkt
- 26: Punkt
- 28: Rotationsachse
- 30: Zylinder
- 32: Nut
- 34: Minimalpunkt
- 36: oberer Bogen
- 38: Schwenkachse
- 40: Hebel
- 42: Nocken
- 44: Außenkontur
- 46: Kurvenscheibe
- 48: Ratschenelement
- 50: Hebel
- 52: Vorsprung
- 54: Ast
- 56: Umschaltelement
- 58: Vertiefung
- 60: Stößel
- 62: Übertragungsscheibe
- 64: Zahnrad
- 66: Antriebsscheibe
- 68: Konturscheibe
- 70: Zahn
- 72: äußerer Zahnbereich
- 74: innerer Zahnbereich
- 76: Zugseil
- 78: Ausnehmung
- 80: Federelement
- 82: Steuerpin
- 84: Zuggurt
- 86: Schulterschlaufe
- 88: Schulter

## Patentansprüche

1. Prothesenhand mit
einem Daumenelement (2) und
wenigstens einem Fingerelement,
wobei das Daumenelement (2) durch eine Steuerkraft (16) relativ zu dem wenigstens einen Fingerelement bewegbar ausgebildet ist,
**dadurch gekennzeichnet, dass**
das Daumenelement (2) in wenigstens einem ersten Modus entlang einer ersten Trajektorie und in wenigstens einem zweiten Modus entlang einer zweiten Trajektorie relativ zu dem wenigstens einen Fingerelement bewegbar ist und von dem ersten Modus in den zweiten Modus bringbar ist, indem die Steuerkraft (16) einen ersten vorbestimmten Grenzwert übersteigt.

2. Prothesenhand nach Anspruch 1, **dadurch gekennzeichnet, dass** das Daumenelement (2) von dem zweiten Modus in den ersten Modus bringbar ist, indem die Steuerkraft (16) einen zweiten vorbestimmten Grenzwert überschreitet, der vorzugsweise identisch zu dem ersten vorbestimmten Grenzwert ist.

3. Prothesenhand nach Anspruch 1 oder 2, **dadurch gekennzeichnet, dass** die Prothesenhand wenigstens ein Kraftaufbringelement (18) aufweist, das eine Rückstellkraft auf das Daumenelement (2) aufbringt, die der Steuerkraft (16) entgegengerichtet ist.

4. Prothesenhand nach Anspruch 3, **dadurch gekennzeichnet, dass** die Rückstellkraft derart gewählt ist, dass sie das Daumenelement (2) ohne Steuerkraft (16) im ersten Modus in eine erste Endposition (A) und im zweiten Modus in eine zweite Endposition (B) bringt, die von der ersten Endposition (A) verschieden ist.

5. Prothesenhand nach Anspruch 4, **dadurch gekennzeichnet, dass** das Daumenelement (2) in der ersten Endposition (A) und/oder in der zweiten Endposition (B) an dem wenigstens einen Fingerelement anliegt.

6. Prothesenhand nach einem der vorstehenden Ansprüche, **dadurch gekennzeichnet, dass** die Prothesenhand wenigstens ein Zugelement (76) aufweist, durch das die Steuerkraft (16) aufbringbar ist.

7. Prothesenhand nach Anspruch 6, **dadurch gekennzeichnet, dass** das wenigstens eine Zugelement (76) ausgebildet ist, an einer Schulter, vorzugsweise der der Prothesenhand gegenüberliegenden Schulter, oder einem Rumpf eines Trägers der Prothesenhand angeordnet zu werden.

8. Prothesenhand nach Anspruch 6 oder 7, **dadurch gekennzeichnet, dass** das Zugelement (76) durch einen Aktuator betätigbar ist.

9. Prothesenhand nach einem der vorstehenden Ansprüche, **dadurch gekennzeichnet, dass** das Daumenelement (2) in wenigstens einem dritten Modus entlang einer dritten Trajektorie und vorzugsweise in wenigstens einem vierten Modus entlang einer vierten Trajektorie bewegbar ist.

## Claims

1. A prosthetic hand with
one thumb element (2) and
at least one finger element,
the thumb element (2) being designed such that it can be moved relative to the finger element by a steering force (16),
**characterised in that**
the thumb element (2) can be moved relative to the at least one finger element along a first trajectory in at least a first mode and along a second trajectory in at least a second mode, and can be brought from the first mode into the second mode by the steering force (16) exceeding a first predetermined limit.

2. The prosthetic hand according to claim 1, **characterised in that** the thumb element (2) can be brought from the second mode into the first mode by the steering force (16) exceeding a second predetermined limit, which is preferably identical to the first predetermined limit.

3. The prosthetic hand according to claim 1 or 2, **characterised in that** the prosthetic hand has at least one force application element (18) that applies a restoring force to the thumb element (2) that opposes the steering force (16).

4. The prosthetic hand according to claim 3, **characterised in that** the restoring force is selected in such a way that in the first mode it moves the thumb element (2) into a first end position (A) without the steering force (16) and in the second mode into a second end position (B) that differs from the first end position (A).

5. The prosthetic hand according to claim 4, **characterised in that** in the first end position (A) and/or in the second position (B) the thumb element (2) rests on the at least one finger element.

6. The prosthetic hand according to one of the preceding claims, **characterised in that** the prosthetic hand has at least one tension element (76) through which the steering force (16) can be applied.

7. The prosthetic hand according to claim 6, **characterised in that** the at least one tension element (76) is designed to be arranged on a shoulder, preferably the opposite shoulder to the prosthetic hand, or a torso of a wearer of the prosthetic hand.

8. The prosthetic hand according to claim 6 or 7, **characterised in that** the tension element (76) can be actuated by an actuator.

9. The prosthetic hand according to one of the preceding claims, **characterised in that** the thumb element (2) can be moved in at least a third mode along a third trajectory and preferably in at least a fourth mode along a fourth trajectory.

## Revendications

1. Main prothétique, comportant
un élément de pouce (2) et
au moins un élément de doigt,
l'élément de pouce (2) étant réalisé de manière à pouvoir être déplacé par rapport audit au moins un élément de doigt par une force de commande (16),
**caractérisée en ce que**
l'élément de pouce (2) est mobile par rapport audit au moins un élément de doigt le long d'une première trajectoire dans au moins un premier mode et le long d'une deuxième trajectoire dans au moins un deuxième mode et peut être amené du premier mode au deuxième mode du fait que la force de commande (16) dépasse une première valeur limite prédéterminée.

2. Main prothétique selon la revendication 1,
**caractérisée en ce que** l'élément de pouce (2) peut être amené du deuxième mode au premier mode du fait que la force de commande (16) dépasse une deuxième valeur limite prédéterminée qui est de préférence identique à la première valeur limite prédéterminée.

3. Main prothétique selon la revendication 1 ou 2,
**caractérisée en ce que** la main prothétique comporte au moins un élément d'application de force (18) qui applique à l'élément de pouce (2) une force de rappel qui est opposée à la force de commande (16).

4. Main prothétique selon la revendication 3,
**caractérisée en ce que** la force de rappel est choisie de manière à amener l'élément de pouce (2) sans force de commande (16) jusque dans une première position extrême (A) dans le premier mode et jusque dans une deuxième position extrême (B), différente de la première position extrême (A), dans le deuxième mode.

5. Main prothétique selon la revendication 4,
**caractérisée en ce que** dans la première position extrême (A) et/ou dans la deuxième position extrême (B), l'élément de pouce (2) est en appui contre ledit au moins un élément de doigt.

6. Main prothétique selon l'une des revendications précédentes,
**caractérisée en ce que** la main prothétique présente au moins un élément de traction (76) permettant d'appliquer la force de commande (16).

7. Main prothétique selon la revendication 6,
**caractérisée en ce que** ledit au moins un élément de traction (76) est réalisé pour être disposé sur une épaule, de préférence sur l'épaule opposée à la main prothétique, ou sur un torse d'un porteur de la main prothétique.

8. Main prothétique selon la revendication 6 ou 7,
**caractérisée en ce que** l'élément de traction (76) peut être actionné par un actionneur.

9. Main prothétique selon l'une des revendications précédentes,
**caractérisée en ce que** l'élément de pouce (2) est mobile le long d'une troisième trajectoire dans au moins un troisième mode et de préférence le long d'une quatrième trajectoire dans au moins un quatrième mode.
